# EUROPÄISCHE PATENTSCHRIFT

(11) **EP 2 116 204 B1**
(45) Veröffentlichungstag und Bekanntmachung des Hinweises auf die Patenterteilung: **05.12.2012**
(21) Anmeldenummer: 09156438.5
(22) Anmeldetag: 27.03.2009
(51) Int. Cl.: A61B 17/68, A61B 17/72, A61B 17/88

(54) **Vorrichtung zur Stabilisierung von Röhrenknochenbrüchen**
Device for stabilising hollow bone breaks
Dispositif de stabilisation de fractures d'os longs

(30) Priorität: 07.04.2008 DE 102008017741
(43) Veröffentlichungstag der Anmeldung: 11.11.2009
(73) Patentinhaber: H&R Spezialfedern GmbH & Co. KG., 57368 Lennestadt (DE)
(72) Erfinder: Labitzke, Reiner, Prof. Dr., Schwerte, 58239 (DE)
(74) Vertreter: Fritz, Edmund Lothar

(56) Entgegenhaltungen:
- EP-A- 0 374 088
- WO-A-97/07744
- DE-A1- 19 743 048
- FR-A- 2 848 411

## Beschreibung

Die vorliegende Erfindung betrifft eine Vorrichtung zur Stabilisierung von Röhrenknochenbrüchen umfassend ein als Wendelstab ausgebildetes, in den Hohlraum eines Röhrenknochens einführbares, in das Mark eindrehbares Stabilisierungselement, welches an mindestens einem Ende eine Einrichtung zur Handhabung aufweist, wobei ein weitgehend steifer Wendelstab (10) mit halikaler Form verwendet wird, der eine Vertikalfederrate aufweist, die so hoch eingestellt ist, im Bereich von 1000-6000 N/mm, dass im wesentlichen keine Längenänderung Δ I in Längsrichtung des Wendelstabs auftritt.

Bei einer typischen aus dem Stand der Technik bekannten Lösung zur Stabilisierung von Röhrenknochen-Schaftfrakturen werden starre Nägel eingebracht. Diese sind entweder kompakt oder hohl und vor deren Einbringung muss zunächst der Markraum ausgebohrt werden. In jedem Fall müssen die Nägel mit großer Kraft in den Markraum eingeschlagen werden. Die im Knocheninneren entstehende Druckerhöhung erzeugt Partikelversprengungen aus Marktrümmern, Fett und Blutbestandteilen, die die Blutgefäße insbesondere in Lunge und Hirn verstopfen können. Außerdem werden die innere Knochen-Textur des im hohen Grade mitstabilisierenden und blutbildenden Knochenmarks und das innere Blutgefäßsystem weitgehend zerstört. Damit wird dem Bakterienwachstum der Boden bereitet und die erhöhte Infektanfälligkeit (Osteomyelitis) der Marknagelung erklärt. Beide Strukturen müssen während der Heilungszeit zusätzlich zum eigentlichen Kallus wieder aufgebaut werden, was die Heilung beträchtlich verzögert. Die Starrheit der Nägel hat oft zusätzliche Einschlagbrüche an der Gegenkortikalis, das heißt der gegenüberliegenden Knochenwand, zur Folge. Durch den Nagel auseinander gedrängte Frakturspalte können nicht zusammen wachsen, so dass erneute Operationen erforderlich werden.

Es werden auch starre Platten zur Fixierung von Knochenbrüchen verwendet. Starre Platten haben ebenso wie Marknägel den Nachteil, dass sie die natürliche Kallusbildung verhindern. Weiterhin besteht die Gefahr einer Falschgelenkbildung (Pseudoarthrose, "non-union") und der Entwicklung einer Knocheninfektion bei verlängerter Heilungszeit mit der hohen Wahrscheinlichkeit eines erneuten Bruches nach Entfernung des Osteosynthesematerials. Der Bruch bleibt somit eine Sollbruchstelle.

Aus der DE 38 35 682 C2 ist eine Vorrichtung der eingangs genannten Art bekannt. Bei dieser bekannten Vorrichtung wird vorgeschlagen, eine Art Schraubenfeder in die Röhre eines Röhrenknochens einzuführen. Diese bekannte Vorrichtung wurde nicht nur zur Stabilisierung von Röhrenknochenbrüchen sondern auch von Gelenken vorgeschlagen. Diese bekannte Schraubenfeder war um ihre Längsachse flexibel und ließ sich bei der Einbringung tordieren, wodurch der Durchmesser der Schraubenfeder aufweitbar oder reduzierbar war. Die Schraubenfeder war daher noch vergleichsweise weich und in Grenzen verformbar, das heißt sie wies noch typische Federeigenschaften auf. In den Ausführungsbeispielen der genannten Druckschrift ist eine solche Schraubenfeder dargestellt, die in einen Oberschenkelknochen eingebracht wurde. Dabei ist die Feder entlang ihrer Längsachse gebogen dargestellt.

In der WO 99/22662 A1 wird ebenfalls ein Wendeldraht als Osteosynthesemittel eingesetzt. Bei dieser bekannten Vorrichtung wird eine Dreipunktverspannung vorgeschlagen, die jedoch nachteilig ist, da schädliche bruchverschiebende Kräfte erzeugt werden. Außerdem ist diese bekannte Vorrichtung nur für subcapitale (direkt unterhalb des Kopfes gelegene) Oberarmbrüche bestimmt und ist nicht für Schaftbrüche vorgesehen. Der hier verwendete Wendeldraht ist noch verhältnismäßig weich und elastisch verformbar. Bei der in den Ausführungsbeispielen dargestellten Stabilisierung einer Oberarmfraktur wird der Wendeldraht in einem Winkel gebogen zu seiner Längsachse dargestellt. Außerdem wird der Wendeldraht seitlich durch ein Bohrloch im Oberarmschaft geführt, womit ein Eingriff in die Knochensubstanz erfolgt.

In der Deutschen Patentschrift DE 908 906 B wird eine federnde Knochenschraube, insbesondere für Schräg- und Längsbrüche beschrieben, die somit nicht vorrangig für den Markraum von Röhrenknochen vorgesehen ist. Diese Knochenschraube wird als längs elastisch bezeichnet. Bei der Einbringung wird die Feder gespannt, indem sie tordiert wird. Danach wird sie in ein gebohrtes Loch des Knochens geschoben und dann entspannt, wodurch die Feder weiter wird, das heißt sie weitet sich radial auf. Diese Schraubenfeder muss folglich ausreichend weiche Federeigenschaften aufweisen, um diese Verformungen zuzulassen. Nachdem die Feder sich unten im Knochen festgesetzt hat, wird die Feder dann wieder gespannt. Auch ist an anderer Stelle in dieser Druckschrift davon die Rede, dass sich die obersten Windungen der Schraubenfeder flach drücken lassen, was ebenfalls auf eine elastische Verformbarkeit schließen lässt.

In der WO 97/07744 A wird ein Anker beschrieben, der dazu dient, Gewebe an einem Knochen zu verankern. Dazu wird ein vergleichsweise steifer Wendelstab von außen in Querrichtung zur Knochenachse durch den Knochen bis in den Markraum gebohrt. Dieser Wendelstab ist kürzer als die Querausdehnung des Knochens und ist in der Regel nur etwa 5 mm bis 20 mm lang. Der Wendelstab weist am einen Ende eine Einrichtung zur Handhabung auf, so dass er in den Knochen eindrehbar ist. Dieser bekannte Wendelstab ist nicht dazu vorgesehen, Röhrenknochenbrüche zu stabilisieren und eignet sich wegen seiner geringen Länge auch nicht dazu.

Die DE 197 43 048 A1 beschreibt eine Vorrichtung zur Stabilisierung von Röhrenknochen mit einem als Wendelstab ausgebildeten Stabilisierurigselement, welches ebenfalls am einen Ende eine Einrichtung zur Handhabung aufweist, um ein Eindrehen in den Knochen zu ermöglichen. Die Länge dieses bekannten Wendelstabs beträgt nur 5 bis 12 cm. Die Vorrichtung ist für spezifische Knochenbrüche vorgesehen, nämlich für proximale Oberarmfrakturen oder distale Oberschenkeltrakturen. Diese bekannte Vorrichtung ist oben so breit, dass sie die Knochenfraktur im Kopfbereich des Knochens erfasst und durchdringt dort die schalenartige Gelankfläche. Aufgrund ihrer geringen Länge eignet sie sich nicht dazu, in den Markraum des Röhrenknochens eingeschraubt zu werden, sondern endet bereits dort, wo der zylindrische Abschnitt des Röhrenknochens beginnt. Außerdem wird hier ein Draht mit einer geringen Drahtstärke verwendet und einem geringen Steigungswinkel, wodurch der Wendelstab elastisch und leicht zu komprimieren ist.

Die Aufgabe der vorliegenden Erfindung besteht darin, einen Wendelstab der eingangs genannten Gattung, der in den Hohlraum des Röhrenknochens und somit in das Mark eingedreht wird und nur einen minimal invasiven operativen Eingriff notwendig macht, zur Verfügung zu stellen, der einerseits mechanisch günstige Eigenschaften ähnlich wie ein starrer Nagel aufweist, andererseits jedoch noch minimale Bewegungen in der Bruchregion zulässt, die die Knochenheilung fördern.

Die Lösung dieser Aufgabe liefert eine Vorrichtung der eingangs genannten Gattung mit den kennzeichnenden Merkmalen des Hauptanspruchs.

Erfindungsgemäß ist vorgesehen, dass ein weitgehend steifer Wendelstab mit helikaler Form verwendet wird, der eine Vertikalfederrate aufweist, die so hoch eingestellt ist, dass im wesentlichen keine Längenänderung Δ I in Längsrichtung des Wendelstabs auftritt.

Die typische Vertikalfederrate eines solchen Wendelstabs liegt bei 1000-6000 N/mm, bevorzugt bei 1500-5000 N/mm, wobei sich diese Werte auf eine Windung des Wendelstabs beziehen. Weiterhin wird die Biegefederrate des Wendelstabs so gewählt, dass die Knochenbildung "dynamisch" gefördert wird.

Bei der natürlichen Knochenheilung entsteht Kallus. Seine Bildung wird gefördert durch kleine kaum merkbare Bewegungen innerhalb der Bruchzone. Die dadurch entstehenden wechselnden Druck- und Zugkräfte aktivieren die Osteoblasten, die knochenaufbauenden Zellen. Steife Implantate behindern diesen Prozess. Daher ist ein quasi "dynamisches" Implantat vorteilhaft zur Förderung der Knochenheilung. Ein völlig steifer Marknagel wie er früher verwendet wurde ist hingegen biologisch ungünstig. Andererseits ist er mechanisch zur Stabilisierung des Knochens günstig, da er in der Achse des Knochens liegt. Die im Stand der Technik beschriebenen Schraubenfedern haben wiederum den Nachteil, dass sie zu weich sind und zu stark federn und somit den Bruch nicht ausreichend mechanisch stabilisieren. Jahrelange Entwicklungen im Rahmen der vorliegenden Erfindung haben gezeigt, dass man einen Wendelstab optimal zur Stabilisierung von Röhrenknochenbrüchen verwenden kann, wenn er ausreichend steif ist (und somit in Längsrichtung nicht federt), dieser aber aufgrund der Wendelform anders als ein herkömmlicher Marknagel noch minimale (physiologische) Bewegungen im sub-mm-Bereich zulässt und somit die Knochenheilung fördert.

Das auf einer solchen wendelförmigen Vorrichtung (auch EndoHelix genannt) beruhende erfindungsgemäße Osteosyntheseprinzip für Schaftfrakturen langer Röhrenknochen hat zu starren Nägeln ein biomechanisch völlig gegensätzliches Konzept: Mit streng limitierter Flexibilität respektive kontrollierter Steifigkeit (die jedoch deutlich unter der eines konventionellen Nagels liegt) wird der Physiologie der Knochenbruchheilung-Kallusbildung durch kleinste Bewegungen-fördernd zugearbeitet. Der erfindungsgemäße Wendelstab ist in Richtung seiner Längsachse praktisch nicht komprimierbar und hat auch eine hohe Widerstandsfähigkeit gegen Biegung. Der Wendelstab hat zwar die hellkale Form einer Schraubenfeder aber nicht die typischen Federeigenschaften und ist in seiner Steifigkeit in etwa vergleichbar mit der Wendel eines Korkenziehers.

Es handelt sich bei dem erfindungsgemäßen Wendelstab um einen aus einem Draht oder Stab beispielsweise durch Kaltverformung in eine Spiralform (Helixform) gebogenen Wendelstab, bei dem die einzelnen Windungen voneinander beabstandet sind. Aufgrund der großen Steifigkeit der Wendel in Achsrichtung (Vertikalfederrate) ist es nicht möglich, durch Druck in Achsrichtung den Wendelstab zu komprimieren. Anders als bei einer Schraubenfeder bleiben somit die Windungen des Wendelstabs beabstandet. Die Helix des erfindungsgemäßen Wendelstabs hat bevorzugt eine vergleichsweise große Steigung. Diese Steigung kann beispielsweise so gewählt sein, dass das vertikale Maß bei einer vollständigen 360 °-Windung der Helix größer ist als der Außendurchmesser der Helix. Für die Anwendung im Operationsbereich gibt es Wendelstäbe mit verschiedenen Außendurchmessern, da verschiedene Größen wegen der unterschiedlichen Abmessungen der Knochen notwendig sind. Ebenso kann die Drahtstärke des Drahts, aus dem die Wendel gebogen ist variieren, womit auch der Innendurchmesser variiert.

Dennoch bleibt eine minimale Restbiegsamkeit quer zur Achse des Wendelstabs die ausreicht, um die gewünschte physiologische Wirkung zu erzielen. Die Biegefederrate ist so gewählt, dass hier Biegungen quer zur Achse des Wendelstabs im Bereich von weniger als 1 mm oder Bruchteilen von Millimetern möglich sind, die für die physiologische Wirkung ausreichen. Der Wendelstab hat die Eigenschaft sich zu strecken (nicht sich axial zu verlängern, sondern sich in Längsrichtung der Knochenachse auszurichten). Dadurch gelingt es, die Knochenachse stabil zu halten, dabei aber gleichzeitig Minimalbewegungen in der Bruchregion zuzulassen, um die Kallusbildung anzuregen. Durch die nur minimale aber physiologisch noch wirksame Flexibilität ist es möglich, das minimale Bewegungsausmaß zur beschleunigten Knochenheilung zu nutzen. Klinische Verlaufskontrollen haben gezelgt, dass eine hervorragende Kallusbildung in vergleichsweise kurzer Zeit möglich ist.

Durch die ausreichende Steifigkeit des Wendelstabs ist es nicht nur möglich, diesen mittels eines geeigneten im oberen Bereich angreifenden Werkzeugs in den Markraum einzudrehen (ohne das Knochenmark zu entfernen), sondern dieser kann auch nach der Heilung durch Drehen in Gegenrichtung wieder entfernt werden. Der Wendelstab ist im Gegensatz zu den bekannten Lösungen nicht tordierbar und wird weder gespannt noch radial aufgeweitet, um ihn im Markraum festzulegen. Einfaches Eindrehen in das Knochenmark genügt, da der Wendelstab aufgrund seiner Elgenstelfigkeit danach quasi selbsttragend im Knochenmark in seiner Position verbleibt. Zur Einbringung kann eine minimalinvasive OP-Technik verwendet werden, da lediglich etwa 3 cm lange Schnitte notwendig sind. Die auf diese Art stabilisierten Brüche heilen wesentlich schneller als bei anderen Verfahren und die Methode ist komplikationsärmer, da der Operationsschaden geringer ist und das blut- und knochenbildende Gewebe erhalten bleibt.

Bislang wurde bei den bekannten Vorrichtungen der aus dem Knochen herausstehende obere Teil der Schraubenfeder gekürzt. Das hatte Schwierigkeiten sowohl beim endgültigen eindrehen als auch beim Ausdrehen der Helix nach der Knochenheilung zur Folge. Der erfindungsgemäße Wendelstab lässt hingegen ein Einsetzen in einem vereinfachten standardisierten Operationsverfahren zu und lässt sich nach der Knochenheilung problemlos wieder herausdrehen.

Gemäß einer bevorzugten Weiterbildung der Erfindung ist vorgesehen, dass der Wendelstab einen genormten einheitlich konfigurierten Kopf aufweist, der von dem variablen an den jeweiligen Knochen angepassten Außendurchmesser des Wendelstabs unabhängig ist und sich an dem Kopf wenigstens ein etwa axial vorstehender Stift befindet, der aus der ersten Windung geformt und mit dieser einstückig verbunden ist. Der Durchmesser des Wendelstabs muss der Größe des Markraums angepasst sein, die, da auch Knochenbrüche bei Kindern stabilisiert werden, innerhalb weiter Bereiche variieren kann. Dies führt dazu, dass der Hersteller ein Sortiment unterschiedlich großer Wendelstäbe für den Operateur zur Verfügung stellen muss. Für jede dieser Größen würde somit bei der Handhabung des Wendelstabs ein jeweils angepasstes Werkzeug notwendig. Dies vermeidet die erfindungsgemäße Weiterbildung durch den in Größe und Form genormten Kopf des Wendelstabs.

Anders als bislang geht man bei der Einbringung der erfindungsgemäßen Vorrichtung nun bevorzugt so vor, dass man nach einer zum Beispiel röntgenologischen Bestimmung der notwendigen Länge vor dem Eindrehen das untere Ende des Wendelstabs mit einem geeigneten Werkzeug (zum Beispiel Bolzenschneider) abtrennt und so diesen auf die benötigte Länge ablängt. Dadurch bleibt der Kopf des Wendelstabs an dessen anderem Ende, welches den Eindrehbereich bildet, erhalten. Mit einem einzigen weiteren Werkzeug kann dann dieser in den Röhrenknochen eingedreht werden, wobei dieses Werkzeug an dem axial vorstehenden Stift angreift. Da der Kopf einheitlich konfiguriert und genormt ist, kann unabhängig von der Größe (Durchmesser) des jeweils bei der Operation verwendeten Wendelstabs ein einheitliches Werkzeug eingesetzt werden. An diesem Stift im Kopfbereich greift man auch an, um den Wendelstab wieder heraus zu drehen.

Gemäß einer möglichen Variante der Aufgabenlösung ist bevorzugt vorgesehen, dass der Wendelstab mindestens über einen Abschnitt seiner Länge einen sich verjüngenden oder sich erweiternden Außendurchmesser aufweist. Beispielsweise kann der Wendelstab unterhalb des Kopfs einen oberen Abschnitt mit sich in Richtung vom Kopfende weg konisch verjüngendem Außendurchmesser aufweisen. Ein solcher beispielsweise etwa trichterförmiger Abschnitt kann zum Beispiel dann vorteilhaft angewendet werden, wenn eine Osteoporose oder stark erweiterte Markräume vorliegen. An den konischen Abschnitt (Trichterabschnitt) kann sich dann bei einem solchen Wendelstab ein zylindrischer Abschnitt mit gleichbleibendem Außendurchmesser anschließen.

Ebenso gut ist es aber auch möglich, dass Außendurchmesser und Steigung des Wendelstabs über dessen Länge konstant sind. Eine solche Ausgestaltungsform kann man zum Beispiel auch verwenden, wenn größere Belastungen neutralisiert werden müssen. In diesem Fall lassen sich beispielsweise zwei dieser helixförmigen Wendelstäbe gleicher Gestaltung ineinanderdrehen, so dass deren Windungen dann jeweils parallel laufen.

Im Vergleich zu den vorbekannten Lösungen wird erfindungsgemäß ein konstruktiv erheblich versteifter Wendelstab verwendet, der bevorzugt eine mit zwischen 1500 und 5000 N/mm so hoch eingestellte Vertikalfederrate aufweist, dass kein Federeffekt in Längsrichtung (keine Längenänderung Δ I) mehr auftritt. Weiter wurde auch die Biegefederrate bevorzugt in einen Bereich abgesenkt, der nur noch "physiologische" Biegeausschläge ermöglicht. Durch diese Biegefederrate wird die Biegung des Wendelstabs quer zu seiner Längserstreckung definiert. Hier ist zu beachten, dass sich der in den Markraum eingeführte Wendelstab anders verhält als ein freiliegender Wendelstab. Der erfindungsgemäße Wendelstab unterscheidet sich jedoch von dem bekannten Marknagel mit extrem hoher Biegefederrate, der überhaupt keine Biegungen mehr zulässt und damit jeden natürlichen Heilungsreiz auf den Bruch unterdrückt. Die Biegefederrate liegt je nach Außendurchmesser des erfindungsgemäßen Wendelstabs bei einer Länge von beispielsweise 300 mm, einseitig eingespannt und am anderen Ende durchgebogen, zwischen 250 und 1250 mN/mm, bevorzugt zwischen 300 und 1250 mN/mm (Millinewton pro mm). Ein herkömmlicher Marknagel hat im Vergleich dazu Biegefederraten zwischen 5000 und 15000 mN/mm. Der Knochen registriert daher den Marknagel als "steifes Bauteil", von dem kein heilungsfördernder Reiz ausgeht.

Gemäß einer bevorzugten Weiterbildung der Erfindung verwendet man einen Wendelstab, der mindestens in Teilbereichen eine aufgerauhte Oberfläche aufweist. Dies erleichtert das Einwachsen der Vorrichtung in den Fällen, in denen diese nicht wieder entfernt werden soll, so dass sie als Dauerstabilisator dient, beispielsweise bei älteren Patienten.

Eine weitere bevorzugte Weiterbildung sieht vor, dass der Wendelstab im Bereich seiner Spitze sowohl an der Innenseite der Wendel als auch an der Außenseite der Wendel schräg angeschliffen ist. Dadurch wird ein physiologisch günstiges selbstschneidendes vorderes Ende geschaffen, wobei beim Eindrehen des Wendelstabs dieses und eventuelle Knochensplitter nach innen hin weggedrückt werden, so dass sie das weitere Eindrehen nicht behindern.

Der erfindungsgemäß verwendete Wendelstab kann zum Beispiel aus Drähten beliebigen Querschnitts bestehen, vorzugsweise mit rundem oder ellipsoiden Querschnitt. Er ist erfindungsgemäß so versteift, dass er nicht mehr zur Stabilisierung von Gelenken geeignet ist. Die Windungen des helikalen Wendelstabs dürfen nicht aufeinander liegen, da dann der Wendelstab zu weich wird. Zuviel Biegung des Wendelstabs führt zu zuviel Bewegung des Bruches und behindert damit die Heilung. Es besteht dann die Gefahr einer Pseudoarthrose und es wäre immer ein zusätzlicher Gipsverband notwendig. Der erfindungsgemäße Wendelstab besteht besonders bevorzugt aus einem speziellen Federstahl, der ein höheres E-Modul hat als zum Beispiel Titan. Die Verwendung von Titan würde einen größeren Außendurchmesser und eine größere Drahtstärke erfordern, die nicht in Betracht kommt, da die Markräume dazu zu eng sind. Beispielsweise ist erfindungsgemäß eine 1.4441-Stahllegierung besonders gut geeignet.

Bei der Schaffung eines Wendelstabs für die Stabilisierung von Röhrenknochenbrüchen gemäß der Erfindung beeinflussen verschiedene Parameter die Eigenschaften wie Vertikalfederrate und Biegefederrate, dies sind insbesondere die Steigung der Wendel, die Drahtstärke, der äußere Durchmesser des Wendelstabs und der Werkstoff, aus dem der Wendelstab besteht. Gegebenenfalls spielt auch das Fertigungsverfahren zur Herstellung der Wendelform durch Verformung aus einem Draht oder Stab eine Rolle. Der äußere Durchmesser variiert abhängig von der Größe des zu stabilisierenden Knochens. Zur Einstellung der Vertikalfederrate können beispielsweise die Drahtstärke und die Steigung variiert werden. Mögliche beispielhafte Abmessungen für einen Wendelstab, der für eine erfindungsgemäße Vorrichtung in Betracht kommt, sind zum Beispiel bei einer Länge von 250 - 300 mm ein Außendurchmesser von 8 - 15 mm, eine Drahtstärke von 2 - 4 mm, ein Innendurchmesser von 4 - 9 mm und eine Steigung von 15 - 20 mm pro Windung.

Die in den Unteransprüchen genannten Merkmale betreffen bevorzugte Weiterbildungen der erfindungsgemäßen Aufgabenlösung. Weitere Vorteile der Erfindung ergeben sich aus der nachfolgenden Detailbeschreibung.

Nachfolgend wird die vorliegende Erfindung anhand von Ausführungsbeispielen unter Bezugnahme auf die beiliegenden Zeichnungen näher beschrieben.

Dabei zeigen:
Figur 1 eine schematisch vereinfachte Darstellung eines Teils einer erfindungsgemäßen Vorrichtung nach einer beispielhaften Ausführungsvariante;
Figur 2 eine schematisch vereinfachte vergrößerte Darstellung, die das Eindrehen des Wendelstabs mittels eines Werkzeugs verdeutlicht;
Figur 3 eine schematisch vereinfachte Darstellung des oberen Teils eines Wendelstabs gemäß einer alternativen Ausführungsvariante der Erfindung;
Figur 5 eine Ansicht eines Hilfsmittels, welches für das Einrichten der verschobenen Bruchenden verwendet wird (Repositionsstab);
Figur 6 eine Ansicht eines weiteren Hilfsmittels (Leitstab), welches vor dem Eindrehen der Schraubenfeder eingebracht wird;
Figur 7 eine Ansicht eines doppelten Wendelstabs, welcher in bestimmten Anwendungsfällen gemäß einer alternativen Variante der Erfindung eingesetzt wird.

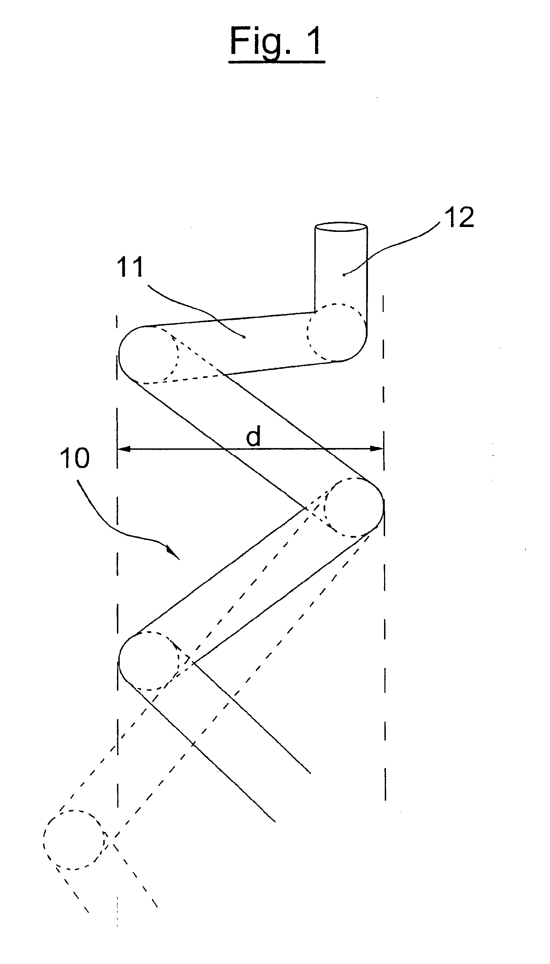

Zunächst wird auf die Figur 1 Bezug genommen. Die Darstellung zeigt in vergrößertem Maßstab den oberen Teil eines erfindungsgemäßen Wendelstabs 10, der bei einem Knochenbruch in den Röhrenknochen eingeschoben wird. Dieser Wendelstab 10 hat einen Außendurchmesser d, der variieren kann, je nach Größe des jeweiligen Röhrenknochens, in den der Wendelstab eingesetzt wird. Die Steigung des Wendelstabs 10 kann ebenfalls je nach Abwendungsfall variieren. Der untere Teil des Wendelstabs 10 ist in der Zeichnung nicht dargestellt, da dieser analog gestaltet ist und sich die Windungen in gleicher Ausgestaltung weiter fortsetzen. Die jeweils notwendige Länge wird vor deren Einsetzen bestimmt und der Wendelstab wird dementsprechend am unteren Ende abgelängt. Der Querschnitt des Drahts, aus dem der Wendelstab 10 in dem Ausführungsbeispiel besteht, ist rund, kann aber auch im Prinzip nahezu beliebig anders gestaltetet sein. Der Wendelstab 10 besteht aus einem Metalldraht einer speziellen Stahllegierung (beispielsweise 1.4441), die sich für medizinische Anwendungen eignet und hat eine hohe Steifigkeit. Es handelt sich also nicht um eine Feder, die wie eine Zugfeder elastisch ist bei Längendehnung und sich dann zurückverformt. Vielmehr ist die Steifigkeit so hoch, dass praktisch keine Längendehnung (oder Komprimierung in Längsrichtung) auftritt.

Wie man aus Figur 1 erkennen kann, ist die oberste Windung 11 im oberen Endbereich (Kopfbereich) des Wendelstabs eine gestauchte Windung, das heißt eine Windung mit ist gegenüber den übrigen Windungen verringerter Steigung. An die oberste gestauchte Windung 11 schließt sich dann ein in axialer Richtung ausgerichteter Stift 12 an, der als Angriffspunkt beim Eindrehen des Wendelstabs mit einem spezifischen Werkzeug dient, wie weiter unten noch näher erläutert werden wird. In diesem Kopfbereich mit der obersten Windung 11 und dem axial ausgerichteten am Federende vorstehenden Stift 12 ist der Wendelstab 10 genormt. Dies bedeutet, dieser Kopfbereich ist immer gleich gestaltet und dimensioniert, unabhängig von dem jeweiligen Außendurchmesser und der Steigung der Schraubenfeder. Die beiden letztgenannten Größen können entsprechend dem Anwendungsfall variieren. Da jedoch der Kopfbereich jeweils gleich gestaltet ist, kann bei der Operation für die Handhabung des Wendelstabs 10 immer ein einheitliches Werkzeug verwendet werden.

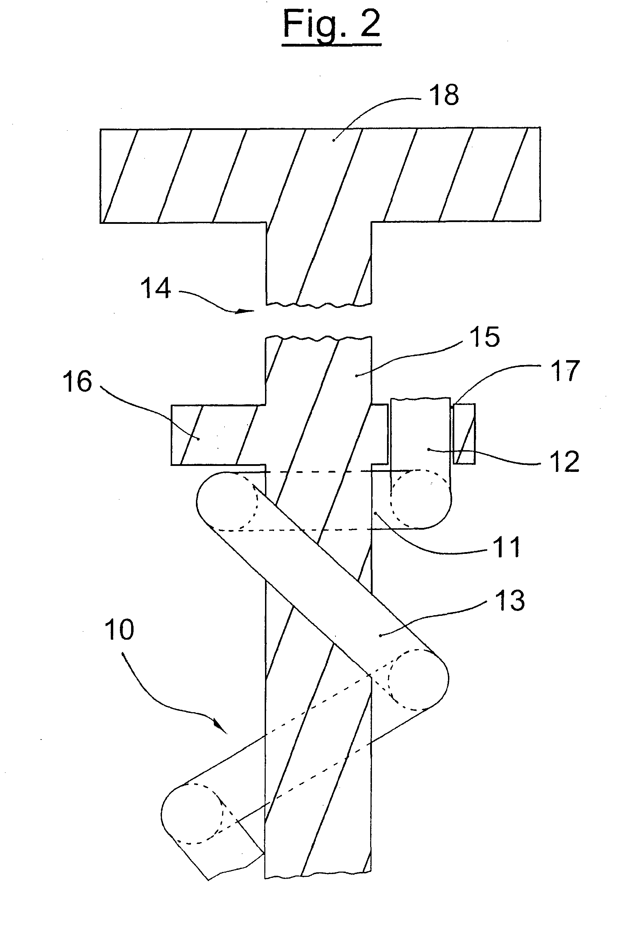

Nachfolgend wird nun unter Bezugnahme auf Figur 2 der Vorgang des Eindrehens des Wendelstabs in den Röhrenknochen bei der Operation näher erläutert. Die Darstellung zeigt den Kopfbereich des Wendelstabs 10 in einem gegenüber Figur 1 etwas vergrößerten Maßstab. Man erkennt die zweitoberste Windung 13 und die endseitige gestauchte Windung 11 mit dem axial vorstehenden Stift 12. Das verwendete Werkzeug 14 hat einen Schaft 15 mit einem beispielsweise plattenartigen Element 16 mit radialer Ausdehnung, welches beispielsweise ringförmig ausgebildet sein kann wie ein Kragen oder auch rechteckig als Platte oder als Querstift. In diesem radialen Element 16 befindet sich eine Bohrung 17 oder Ausnehmung, in die der axial vorstehende Stift 12 des Wendelstabs eingeschoben werden kann. Gleichzeitig dient der untere Abschnitt des Schafts 15 als Zentriermittel für das Werkzeug 14, da er wie man sieht koaxial im Kopfbereich in den Wendelstab 10 eingreift. Am Ende des Schafts 15 des Werkzeugs 14 befindet sich ein Handgriff 18, so dass man dort angreifen kann, um das Werkzeug 14 um seine Achse zu drehen. Dabei wird der Stift 12 mitgenommen und folglich der Wendelstab 10 mitgedreht. Bei der Entfernung kann man das gleiche Werkzeug 14 benutzen und durch Drehung in umgekehrter Richtung des Wendelstabs wieder aus dem Knochen herausdrehen.

Anstelle der Drehung des Werkzeugs 14 von Hand kann es vorteilhaft sein, dieses über eine maschinelle Vorrichtung zu bedienen, die geeignet ist, das Werkzeug mit vergleichsweise geringer Umdrehungszahl zu drehen. In diesem Fall sind hier nicht dargestellte Kupplungsmittel oben am Kopfbereich 18 des Werkzeugs 14 vorgesehen, die mit entsprechenden Kupplungsmitteln an der maschinellen Eindrehvorrichtung zusammenwirken, zum Beispiel kann dies ein Sechskant, Imbus oder eine andere Angriffsfläche am Werkzeug sein. Diese Variante hat gegenüber der Handbetätigung den Vorteil, dass man nicht nach jeder halben Drehung neu am Werkzeug angreifen muss, wodurch der Eindrehvorgang unterbrochen würde, was gegebenenfalls nachteilig ist.

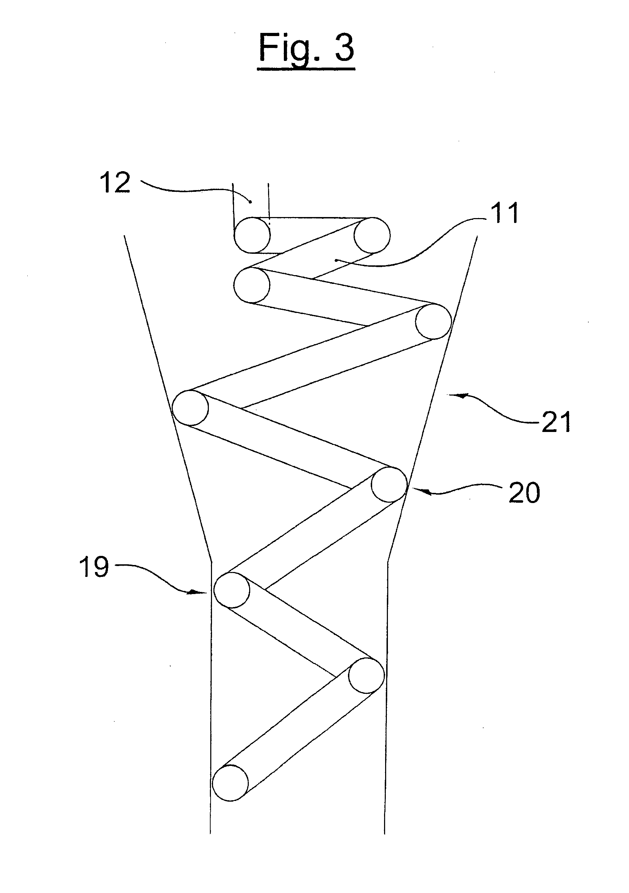

Nachfolgend wird nun unter Bezugnahme auf die Figur 3 ein alternatives Ausführungsbeispiel der vorliegenden Erfindung näher erläutert. Es ist eine Variante des Wendelstabs 20 dargestellt, bei der anders als in dem zuvor beschriebenen Ausführungsbeispiel der Außendurchmesser des Wendelstabs in dem oberen Bereich nicht konstant ist. Es ist hier ein zylindrischer Abschnitt 19 vorhanden, an den sich axial im oberen Bereich ein sich im Außendurchmesser konisch nach oben zum Kopfende des Wendelstabs hin erweiternder Trichterabschnitt anschließt. Somit nimmt der Durchmesser des Wendelstabs in diesem Trichterabschnitt 21 zum Kopfende hin allmählich zu, was bei bestimmten Anwendungen wie zum Beispiel bei erweiterten Markräumen vorteilhaft ist. Auf den Trichterabschnitt 21 folgt dann auch hier wieder der genormte Kopfbereich mit der gestauchten Windung 11 und dem axial ausgerichteten Stift 12, so dass die Handhabung des Wendelstabs 20 analog zu dem bereits beschriebenen Wendelstab 10 gemäß dem Ausführungsbeispiel der Figuren 1 und 2 erfolgen kann.

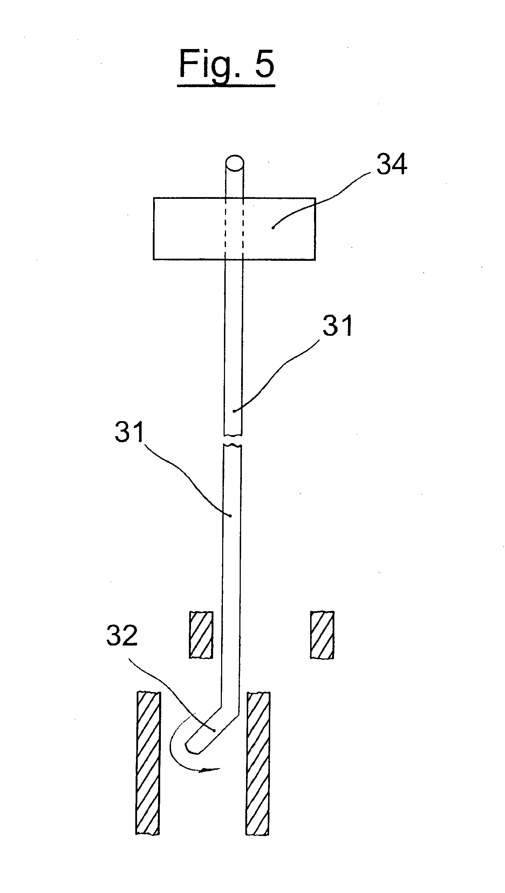

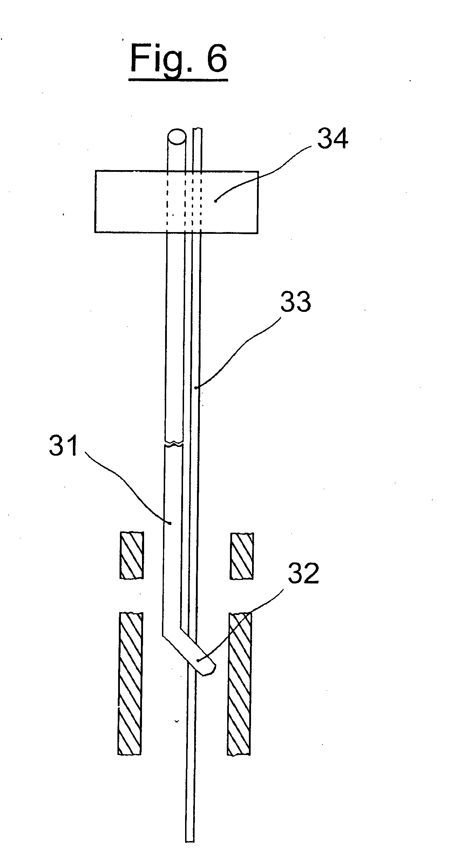

Nachfolgend werden unter Bezugnahme auf die Figuren 5 und 6 spezifische Werkzeuge erläutert, die man bei der Positionierung eines erfindungsgemäßen Wendelstabs verwenden sollte. Figur 5 zeigt einen so genannten Repositionsstab 31, den man für das Einrichten der verschobenen Bruchenden verwendet. Es handelt sich um einen länglichen Stab beispielsweise aus Stahl, der an seiner Spitze eine Abwinklung 32 aufweist. Er wird vor dem Einbringen des Wendelstabs 10 unter drehender Führung bis ins untere Knochenbruchstück vorgeschoben. Wenn die Einrichtung unter Drehen des Repositionsstabs 31 geglückt ist, wird ein zweiter dünnerer so genannter Leitstab 33, der in Figur 6 dargestellt ist und ebenfalls in der Regel aus Edelstahl besteht, neben dem Repositionsstab 31 bis ins untere Fragment eingebracht. Dieser Leitstab dient quasi als Führung für den Wendelstab 10, die nur über diesen Leitstab 33 eingedreht werden sollte. Dies dient dazu, zu garantieren, dass es beim Einführen des Wendelstabs nicht zur Verletzung von Nerven oder Gefäßen kommt. Beide Stäbe 31, 33 können jeweils mit einem Handgriff 34, 35 geführt werden. Die Skizze gemäß Figur 5 verdeutlicht, wie das untere Ende mit der Abwinklung 32 die verschobenen Bruchenden richtet. Figur 6 verdeutlicht die relative Position der beiden Stäbe 31 und 33 nach deren Einführung in den Röhrenknochen vor dem Eindrehen des Wendelstabs.

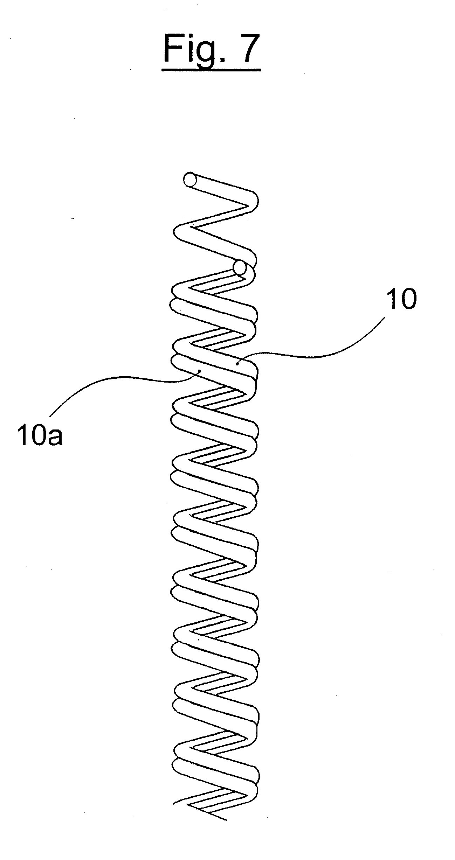

Nachfolgend wird unter Bezugnahme auf die Figur 7 ein weiteres alternatives Ausführungsbeispiel der vorliegenden Erfindung näher erläutert. Bei dieser Variante wird ein doppelter Wendelstab angewandt, um beispielsweise größere Belastungen zu neutralisieren. Wenn man einen ersten Wendelstab 10 beispielsweise mit einer Ausgestaltung wie sie zuvor bereits anhand von Figur 1 beschrieben wurde, verwendet und dann in diesen einen gleich gestalteten zweiten Wendelstab 10 a mit gleichem Außendurchmesser und gleicher Steigung hineindreht, erhält man einen Doppelstab wie er in Figur 7 dargestellt ist. Beide Wendelstäbe liegen quasi ineinander, so dass ihre Windungen jeweils parallel verlaufen. Der damit erhaltene Doppelstab besitzt eine doppelte Steifigkeit gegenüber einer einzelnen Wendel, was die genannte Möglichkeit schafft, größere Belastungen aufzunehmen und die Nachgiebigkeit des Wendelstabs bei Bewegungen des Knochens weiter herabsetzt.

Es gibt verschiedene alternative Werkzeuge, die ebenfalls dazu verwendet werden können, einen erfindungsgemäßen Wendelstab in einen Röhrenknochen einzudrehen. Beispielsweise kann man das Ende der obersten Windung des Wendelstabs nach radial einwärts biegen Ein entsprechend geformtes Werkzeug, welches an die Form sich dann ergebenden Hohlraums (C-Form) im Inneren der Wendel angepasst ist, kann dann in diesen Hohlraum in der Windung formschlüssig eingreifen, so dass sich ein Dreheingriff ergibt.

Alternativ kann man beispielsweise auch den Wendelstab im Kopfbereich außen so anschleifen, dass sich eine Vierkant- oder Mehrkantform ergibt. Mit einem entsprechend geformten Werkzeug in Form einer Art Schlüssels mit einem Innenvierkant oder -mehrkant kann man dann über den Kopf der Wendel greifen und so einen Dreheingriff herstellen.

Man kann auch beispielsweise auf eine oberste abgeflachte Windung des Wendelstabs einen Körper aufschweißen, der beispielsweise eine Mehrkantform aufweist, so das man dann mit einem Imbusschlüssel über diesen Mehrkant greifen und die Wendel drehen kann.

Alternativ ist es auch beispielsweise möglich, auf einen spezifisch geformten Kopf an der Wendel zu verzichten und einen oberen Abschnitt der Wendel außen mit einem Werkzeug zu übergreifen, welches eine Art Bohrfutter aufweist, das radial verstellbar ist und so den oberen Abschnitt der Wendel klemmend erfasst.

### Bezugszeichenliste

- 10: Wendelstab
- 10 a: zweiter Wendelstab
- 11: gestauchte Windung
- 12: Stift
- 13: zweitoberste Windung
- 14: Werkzeug
- 15: Schaft
- 16: radiales Element
- 17: Bohrung
- 18: Handgriff
- 19: zylindrischer Teil
- 20: Wendelstab
- 21: Trichterteil
- 31: Repositionsstab
- 32: Abwinklung
- 33: Leitstab
- 34: Handgriff
- 35: Handgriff

## Patentansprüche

1. Vorrichtung zur Stabilisierung von Röhrenknochenbrüchen umfassend ein als Wendelstab ausgebildetes, in den Hohlraum eines Röhrenknochens einführbares, in das Mark eindrehbares Stabilisierungselement, welches an mindestens einem Ende eine Einrichtung zur Handhabung aufweist, wobei ein weitgehend steifer Wendelstab (10) mit helikaler Form verwendet wird, der eine Vertikalfederrate aufweist, die so hoch eingestellt ist, im Bereich von 1000-6000 N/mm, dass im wesentlichen keine Längenänderung ΔI in Längsrichtung des Wendelstabs auftritt, **dadurch gekennzeichnet, dass** der Wendelstab eine Länge von 250 mm bis 300 mm aufweist, geeignet ist, sich in Längsrichtung der Knochenachse auszurichten und dass der Durchmesser des Wendelstabs der Größe des Markraums angepasst ist,

2. Vorrichtung nach Anspruch 1, **dadurch gekennzeichnet, dass** der Wendelstab (10) einen genormten einheitlich konfigurierten Kopf aufweist, der im Durchmesser von dem variablen an den jeweiligen Knochen angepassten Außendurchmesser des Wendelstabs unabhängig ist, wobei im Bereich dieses Kopfs der Wendelstab über den Umfang wenigstens einer Windung eine geringere Steigung aufweist.

3. Vorrichtung nach Anspruch 2, **dadurch gekennzeichnet, dass** sich an dem Kopf wenigstens ein etwa axial vorstehender Stift (12) befindet, der aus der ersten Windung (11) geformt und mit dieser einstückig verbunden ist.

4. Vorrichtung nach Anspruch 2 oder 3, **dadurch gekennzeichnet, dass** der Wendelstab (20) unterhalb des Kopfs einen oberen Abschnitt (21) mit sich in Richtung vom Kopfende weg konisch verjüngendem Außendurchmesser aufweist.

5. Vorrichtung nach Anspruch 4, **dadurch gekennzeichnet, dass** sich an den oberen Abschnitt, den Trichterabschnitt (21), ein weiterer Abschnitt mit im wesentlichen gleich bleibenden Außendurchmesser, der Zylinderabschnitt (19), anschließt..

6. Vorrichtung nach einem der Ansprüche 1 bis 4, **dadurch gekennzeichnet, dass** der Wendelstab (10, 20) eine Vertikalfederrate aufweist, die im Bereich von 1500-5000 N/mm liegt.

7. Vorrichtung nach einem der Ansprüche 1 bis 6, **dadurch gekennzeichnet, dass** der Wendelstab (10, 20) eine solche Biegefederrate aufweist, dass bei einer Länge des Wendelstabs von 300 mm dessen Biegefederrate zwischen 250 und 1250 mN/mm, bevorzugt zwischen 300 und 1250 mN/mm, liegt.

8. Vorrichtung nach einem der Ansprüche 1 bis 5, **dadurch gekennzeichnet, dass** der Wendelstab (10, 20) mindestens in Teilbereichen eine aufgerauhte Oberfläche aufweist.

9. Vorrichtung nach einem der Ansprüche 1 bis 8, **dadurch gekennzeichnet, dass** der Wendelstab in seinem vorderen Endbereich eine selbstschneidende Spitze aufweist, gebildet durch einen jeweils schrägen Anschliff oder Anschnitt des Wendelstabs sowohl innenseitig als auch außenseitig.

## Claims

1. A device for stabilizing long tubular bone fractures, comprising a stabilizing element, which is designed as a spiral rod that can be inserted into the cavity in a tubular bone and is screwed into the marrow; on at least one end, this stabilizing element has a device such that a mostly rigid spiral rod (10) having a helical shape is used, having a vertical spring rate which is set so high, namely in the range of 1000-6000 N/mm, that there is essentially no change in length Δl in the longitudinal direction of the spiral rod, **characterized in that** the spiral rod has a length of 250 mm to 300 mm and is suitable for orienting itself in the longitudinal direction of the axis of the bone, and the diameter of the spiral rod is adapted to the size of the medullary space.

2. The device according to Claim 1, **characterized in that** the spiral rod (10) has a standardized, uniformly configured head, which is independent in diameter of the variable outside diameter of the spiral rod adapted to the respective bone, such that there is a smaller inclination in the area of this head of the spiral rod over the circumference of at least one winding.

3. The device according to Claim 2, **characterized in that** at least one pin (12), which protrudes somewhat axially, is shaped from the first winding (11) and is connected to the winding in one piece is provided on the head.

4. The device according to Claim 2 or 3, **characterized in that** the spiral rod (20) has an upper section (21) beneath the head, said upper section having an outside
diameter which tapers conically in the direction away from the head end.

5. The device according to Claim 4, **characterized in that** another section with essentially a uniform outside diameter, i.e., the cylinder section (19), is connected to the upper section, i.e., the funnel section (21).

6. The device according to any one of Claims 1 to 4, **characterized in that** the spiral rod (10, 20) has a vertical spring rate in the range of 1500-5000 N/mm.

7. The device according to any one of Claims 1 to 6, **characterized in that** the spiral rod (10, 20) has a flexible spring rate such that its flexible spring rate at a 300 mm length of the spiral rod is between 250 and 1250 mN/mm, preferably between 300 and 1250 mN/mm.

8. The device according to any one of Claims 1 to 5, **characterized in that** the spiral rod (10, 20) has a roughened surface in at least some partial areas.

9. The device according to any one of Claims 1 to 8, **characterized in that** the spiral rod has a self-cutting tip in its front end area, formed by an oblique grind and cut of the spiral rod on both the inside and outside.

## Revendications

1. Dispositif de stabilisation de fractures d'os tubulaires, comprenant un élément de stabilisation réalisé sous forme d'une baguette spiralée, pouvant être introduit dans la cavité d'un os tubulaire et pouvant être vissé dans la moelle et qui présente à au moins un bout un système de manipulation, étant utilisée une baguette spiralée largement rigide (10) de forme hélicoïdale qui présente un taux d'élasticité verticale paramétré à un niveau tel que, dans la plage de 1000 à 6000 N/mm, sensiblement aucune modification de longueur Δl ne se produit dans le sens longitudinal de la baguette spiralée, **caractérisé en ce que** la baguette spiralée a une longueur de 250 à 300 mm et est apte à s'orienter dans le sens longitudinal de l'axe de l'os et que le diamètre de la baguette spiralée est adapté à la taille de l'espace occupé par la moelle.

2. Dispositif selon la revendication 1, **caractérisé en ce que** la baguette spiralée (10) présente une tête normalisée à conformation homogène dont le diamètre est indépendant du diamètre extérieur variable adapté à l'os respectif de la baguette spiralée, la baguette spiralée présentant une pente plus faible au niveau de cette tête au-dessus de la circonférence d'au moins une spire.

3. Dispositif selon la revendication 2, **caractérisé en ce que**, au niveau de la tête, se trouve au moins une tige (12) proéminente à peu près axialement qui est formée à partir de la première spire (11) et est reliée à celle-ci en formant un bloc.

4. Dispositif selon la revendication 2 ou 3, **caractérisé**
**en ce que** la baguette spiralée (20) présente en-dessous de la tête une section supérieure (21) dotée d'un diamètre extérieur se rétrécissant en cône en direction du bout de la tête.

5. Dispositif selon la revendication 4, **caractérisé en ce qu'**à la section supérieure, la section en entonnoir (21), se raccorde une autre section dotée d'un diamètre restant sensiblement égal, la section cylindrique (19).

6. Dispositif selon une des revendications 1 à 4, **caractérisé en ce que** la baguette spiralée (10, 20) présente un taux d'élasticité verticale qui se situe dans la plage de 1500 à 5000 N/mm.

7. Dispositif selon une des revendications 1 à 6, **caractérisé en ce que** la baguette spiralée (10, 20) présente un taux d'élasticité à la flexion tel que, avec une longueur de la baguette spiralée de 300 mm, son taux d'élasticité à la flexion se situe dans la plage de 250 à 1250 mN/mm, de préférence de 300 à 1250 mN/mm,

8. Dispositif selon une des revendications 1 à 5, **caractérisé en ce que** la baguette spiralée (10, 20) présente au moins par endroits une surface rugueuse.

9. Dispositif selon une des revendications 1 à 8, **caractérisé en ce que** la baguette spiralée présente dans sa partie terminale avant une pointe autocoupante formée par un meulage respectivement ou une incision oblique de la baguette spiralée aussi bien à l'intérieur qu'à l'extérieur.
